# EUROPEAN PATENT APPLICATION

(11) **EP 2 433 661 A1**
(43) Date of publication of application: **28.03.2012**
(21) Application number: 11008893.7
(22) Date of filing: 21.05.2004
(51) Int. Cl.: A61L 31/16, A61F 2/90

(54) **Anti-restenotic agents to be delivered from a stent**

(30) Priority: 28.05.2003 US 447587; 11.02.2004 US 777881
(62) Divisional of application: 04753187.6
(71) Applicant: Conor Medsystems, Inc., Menlo Park, CA 94025 (US)
(72) Inventor: Parker, Theodore L., Danville, CA 94506 (US); Shanley, John F., Emerald Hils, CA 94062-4072 (US); Trauthen, Brett, Newport Beach, CA 92660 (US); Litvack, Frank, Los Angeles, CA 90077 (US); Diaz, Stephen H., Palo Alto, CA 94301 (US)
(74) Representative: Diehl & Partner GbR

(57) **Abstract**

The invention discloses an anti-restenotic drug to be delivered from a stent loaded with a predetermined amount of the anti-restenotic drug for use in a method of reducing restenosis, the method comprising implanting the stent having no more than the predetermined amount of the anti-restenotic drug; and delivering the anti-restenotic drug from a bioresorbable polymer matrix reservoir in the stent, wherein the polymer matrix is completely resorbed between 30 days and 90 days, inclusive, and the entire predetermined amount elutes from the stent before the polymer matrix is resorbed.

## Description

### BACKGROUND

Most coronary artery-related deaths are caused by atherosclerotic lesions which limit or obstruct coronary blood flow to heart tissue. To address coronary artery disease, doctors often resort to percutaneous transluminal coronary angioplasty (PTCA) or coronary artery bypass graft (CABG). PTCA is a procedure in which a small balloon catheter is passed down a narrowed coronary artery and then expanded to re-open the artery. The major advantage of angioplasty is that patients in which the procedure is successful need not undergo the more invasive surgical procedure of coronary artery bypass graft. A major difficulty with PTCA is the problem of post-angioplasty closure of the vessel, both immediately after PTCA (acute reocclusion) and in the long term (restenosis).

Coronary stents are typically used in combination with PTCA to reduce reocclusion of the artery. Stents are introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient

While the exact mechanisms of restenosis are still being determined, certain agents have been demonstrated to reduce restenosis in humans. One example of an agent which has been demonstrated to reduce restenosis when delivered from a stent is paclitaxel, a well-known compound that is commonly used in the treatment of cancerous tumors. However, many of the stents which are currently under development for delivery of anti-restenotic agents have suboptimal agent release profiles and side effects. In one example, over 90 % of the total agent loaded onto the stent is permanently retained in a thin coating on the surface of the stent and is never delivered to the tissue.

### SUMMARY OF THE INVENTION

The present invention relates to a method for decreasing restenosis following stenting by administration of an anti-restenotic agent in a controlled drug release profile which increases the therapeutic effectiveness of administration. The present invention also relates to a stent having a dosage of anti-restenotic agent affixed thereto for controlled release of the agent at a programmed drug delivery profile.

In accordance with one aspect of the invention, a method of reducing restenosis is provided, wherein the method involves providing a drug delivery stent having a dosage of paclitaxel for delivery to an artery, the dosage arranged such that substantially all the paclitaxel is releasable from the stent upon implantation of the stent in the artery. The method further involves implanting the stent within an artery of a patient; and delivering paclitaxel from the stent to the artery at a minimum release rate of 1 percent of the total dosage of paclitaxel on the stent per day throughout an entire administration period from the time of implantation of the stent until the time that substantially all the paclitaxel is released from the stent.

In accordance with another aspect of the invention, a method of reducing restenosis is provided, wherein the method involves providing a drug delivery stent having a dosage of paclitaxel for delivery to an artery. The method further involves implanting the stent within an artery of a patient; and delivering paclitaxel from the stent to the artery at a substantially linear release rate over an entire period from day one after implantation through day twenty five after implantation, wherein the amount of paclitaxel delivered during the period is at least 25% of the drug loaded on the stent.

In accordance with an additional aspect of the invention, a method of reducing restenosis is provided, wherein the method involves providing a drug delivery stent having a dosage of paclitaxel for delivery to an artery. The method further involves implanting the stent within an artery of a patient; and delivering paclitaxel from the stent to the artery, wherein at least 80% of the entire dosage of paclitaxel provided by the stent is delivered to the artery within 60 days of implantation.

In accordance with a further aspect of the invention, a method of reducing restenosis is provided, wherein the method involves a drug delivery stent having a dosage of an anti-restenotic drug for delivery to an artery, the dosage arranged such that substantially all the drug is releasable from the stent upon implantation of the stent in the artery. The method further involves implanting the stent within an artery of a patient; and delivering the drug from the stent to the artery at a minimum release rate of 1 percent of the total dosage of the drug on the stent per day throughout an entire administration period from the time of implantation of the stent until the time that substantially all the drug is released from the stent, wherein the release rate of the drug is substantially linear from at least day two through day 25.

In accordance with a further aspect of the invention, a method of treating a patient is provided, wherein the method involves providing a drug delivery stent having a dosage of therapeutic agent for delivery to an artery, the dosage arranged such that substantially all the agent is releasable from the stent upon implantation of the stent in the artery. The method further involves implanting the stent within an artery of a patient; and delivering the agent from the stent to the artery at a minimum release rate of 1 percent of the total dosage of the agent on the stent per day throughout an entire administration period from the time of implantation of the stent until the time that substantially all the drug is released from the stent, wherein the release rate of the drug after day one is substantially linear from at least day 2 through day 25.

In accordance with a further aspect of the invention, a stent for reducing restenosis is provided, wherein the stent includes a drug delivery stent having initial unexpanded diameter for insertion of the stent into a coronary artery and an expanded diameter for implantation within a coronary artery. The stent further includes a dosage of paclitaxel for delivery to an artery, the dosage arranged such that substantially all the paclitaxel is releasable from the stent upon implantation of the stent in the artery. Furthermore, the dosage of paclitaxel is arranged to be released at a minimum release rate of 1 percent of the total dosage of paclitaxel on the stent per day throughout an entire administration period from the time of implantation of the stent until the time that substantially all the paclitaxel is released from the stent.

### BRIEF DESCRIPTION OF THE DRAWINGS

The invention will now be described in greater detail with reference to the preferred embodiments illustrated in the accompanying drawings, in which like elements bear like reference numerals, and wherein:
FIG. 1 is a perspective view of one example of a stent according to the present invention.
FIG. 2 is a side view of a portion of the stent of FIG. 1.
FIG. 3 is a side cross sectional view of an example of an opening in a stent showing a matrix with a therapeutic agent and a barrier layer.
FIG. 4 is a side cross sectional view of another example of an opening in a stent showing a matrix with a therapeutic agent.
FIG. 5 is a graph of the cumulative release of paclitaxel from a stent for three different substantially linear release profiles.

### DETAILED DESCRIPTION

A method for decreasing the level of restenosis following a stent placement medical intervention involves the continuous administration of a dose of an anti-restenotic agent or drug from the stent to vascular tissue in need of treatment in a controlled, extended, and substantially linear drug release profile. It is envisioned that the vascular tissue in need of treatment is arterial tissue, specifically coronary arterial tissue. The method of substantially linear extended release increases the therapeutic effectiveness of administration of a given dose of anti-restenotic agent and reduces side effects.

In one example described in detail herein the agent or drug will be contained in reservoirs in the stent body prior to release. In the reservoir example, the drug will be held within the reservoirs in the stent in a drug delivery matrix comprised of the drug and a polymeric material and optionally additives to regulate the drug release. Preferably the polymeric material is a bioresorbable polymer

The following terms, as used herein, shall have the following meanings:
The terms "drug" and "therapeutic agent" are used interchangeably to refer to any therapeutically active substance that is delivered to a living being to produce a desired, usually beneficial, effect.

The term "matrix" or "biocompatible matrix" are used interchangeably to refer to a medium or material that, upon implantation in a subject, does not elicit a detrimental response sufficient to result in the rejection of the matrix. The matrix may contain or surround a therapeutic agent, and/or modulate the release of the therapeutic agent into the body. A matrix is also a medium that may simply provide support, structural integrity or structural barriers. The matrix may be polymeric, non-polymeric, hydrophobic, hydrophilic, lipophilic, amphiphilic, and the like. The matrix may be bioresorbable or non-bioresorbable.

The term "bioresorbable" refers to a matrix, as defined herein, that can be broken down by either chemical or physical process, upon interaction with a physiological environment. The matrix can erode or dissolve. A bioresorbable matrix serves a temporary function in the body, such as drug delivery, and is then degraded or broken into components that are metabolizable or excretable, over a period of time from minutes to years, preferably less than one year, while maintaining any requisite structural integrity in that same time period.

The term "openings" includes both through openings and recesses.

The term "pharmaceutically acceptable" refers to the characteristic of being non-toxic to a host or patient and suitable for maintaining the stability of a therapeutic agent and allowing the delivery of the therapeutic agent to target cells or tissue.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{w} greater than about 3000 and preferably greater than about 10,000 and a M_{w} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.

The term "primarily" with respect to directional delivery, refers to an amount greater than about 50% of the total amount of therapeutic agent provided to a blood vessel.

The term "restenosis" refers to the renarrowing of an artery following an angioplasty procedure which may include stenosis following stent implantation.

The term "substantially linear release profile" refers to a release profile defined by a plot of the cumulative drug released versus the time during which the release takes place in which the linear least squares fit of such a release profile plot has a correlation coefficient, r² (the square of the correlation coefficient of the least squares regression line), of greater than 0.92 for data time points after the first day of delivery. A substantially linear release profile is clinically significant in that it allows release of a prescribed dosage of drug at a uniform rate over an administration period. This controlled release can be essential to staying within the toxic / therapeutic window for a particular drug.

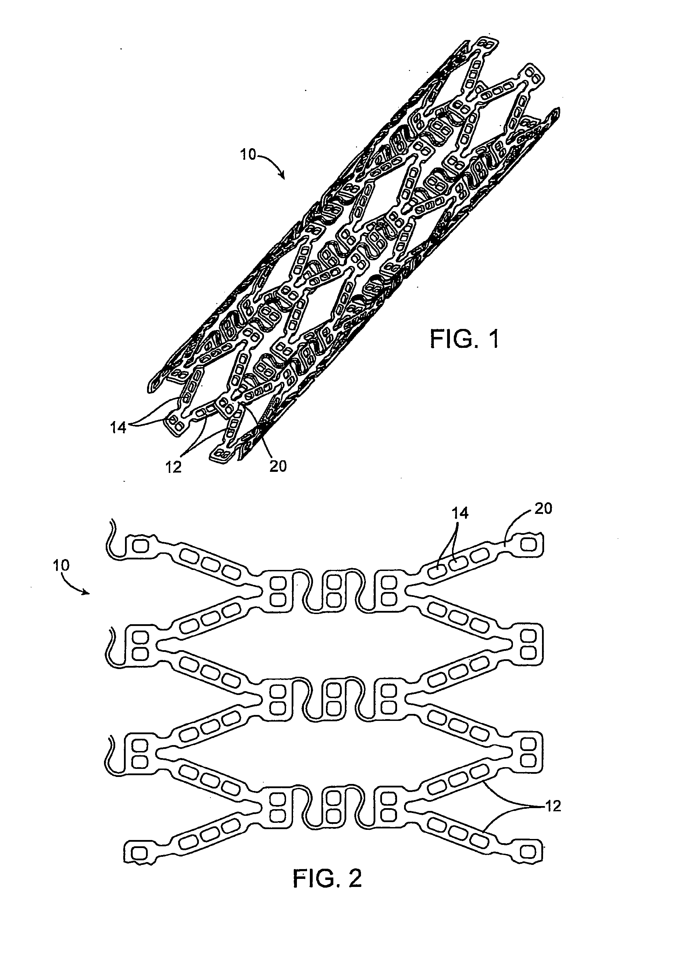

FIG. 1 illustrates one example of an implantable medical device in the form of a stent 10. FIG. 2 is an enlarged flattened view of a portion of the stent of FIG. 1 illustrating one example of a stent structure including struts 12 interconnected by ductile hinges 20. The struts 12 include openings 14 which can be non-deforming openings containing a therapeutic agent. One example of a stent structure having non-deforming openings is shown in U.S. Patent No. 6,562,065 which is incorporated herein by reference in its entirety.

The implantable medical devices of the present invention are configured to release at least one therapeutic agent from a matrix affixed to the implantable body. The matrix is formed such that the distribution of the agent in the polymer matrix directly controls the rate of elution of the agent from the matrix.

In one embodiment, the matrix is a polymeric material which acts as a binder or carrier to hold the agent in or on the stent and/or modulate the release of the agent from the stent. The polymeric material can be a bioresorbable or a non-bioresorbable material.

The therapeutic agent containing matrix can be disposed in the stent or on surfaces of the stent in various configurations, including within volumes defined by the stent, such as openings, holes, or concave surfaces, as a reservoir of agent, or arranged in or on all or a portion of surfaces of the stent structure. When the therapeutic agent matrix is disposed within openings in the strut structure of the stent to form a reservoir, the openings may be partially or completely filled with matrix containing the therapeutic agent.

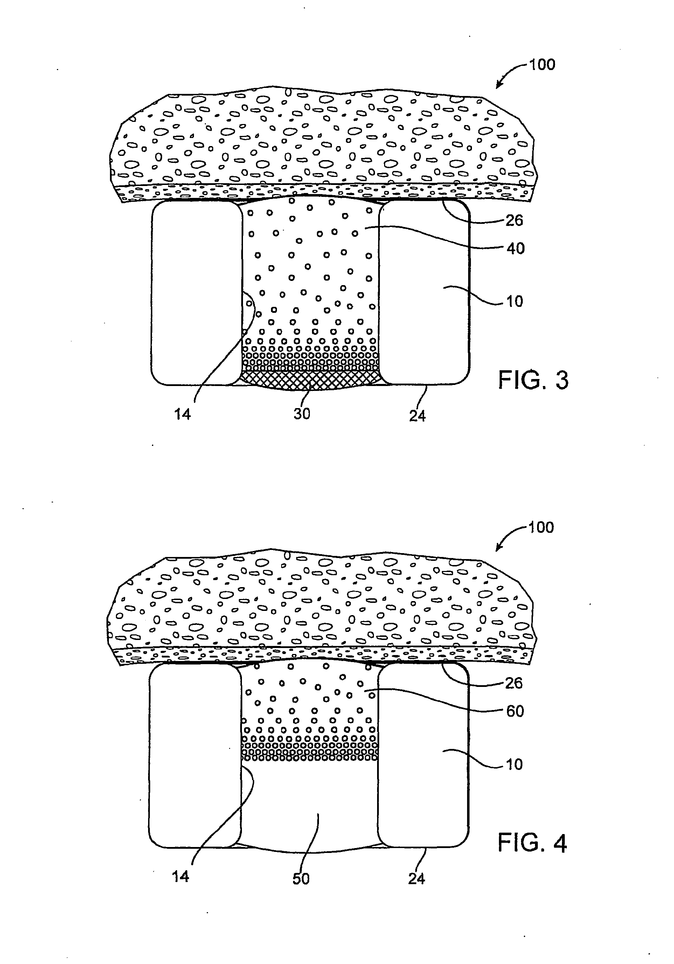

FIG. 3 is a cross section of one strut of the stent 10 and blood vessel 100 illustrating one example of an opening 14 arranged adjacent the vessel wall with a mural surface 26 abutting the vessel wall and a luminal surface 24 opposite the mural surface. The opening 14 of FIG. 3 contains a matrix 40 with a therapeutic agent illustrated by Os in the matrix. The luminal side 24 of the stent opening 14 is provided with a barrier layer 30. The barrier layer 30 erodes more slowly than the matrix 40 containing the therapeutic agent and thus, causes the therapeutic agent to be delivered primarily to the mural side 26 of the stent. The matrix 40 and therapeutic agent are arranged in a programmable manner to achieve a desire release rate and administration period which will be described in further detail below. As can be seen in the example of FIG. 3, the concentration of the therapeutic agent (Os) is highest at the luminal side 24 of the stent 10 and lowest at the mural side 26 of the stent. This configuration in which the drug can be precisely arranged within the matrix allows the release rate and administration period to be selected and programmed to a particular application. The methods by which the drug can be precisely arranged within the matrix in the openings is a stepwise deposition process is further described in U.S. Patent Application Serial No. 10,777,283 filed on February 11, 2004, and is incorporated herein by reference.

FIG. 4 is a cross section of another example of an opening 14 in a stent 10 containing a matrix and therapeutic agent. The opening 14 of FIG. 4 contains a matrix with a therapeutic agent illustrated by Os in the matrix. The portion of the matrix 50 located at the luminal ¼ to ¾ of the stent opening 14 includes matrix without the anti-restenotic agent while the portion of the matrix 60 located at the mural ¼ to ¾ of the stent opening includes matrix with anti-restenotic agent. Preferably, the matrix with anti-restenotic agent 60 is located in about the mural ½ of the stent opening. An arrangement with the anti-restenotic agent positioned closer to the mural side 26 of the stent achieves directional delivery of the anti-restenotic agent primarily to the mural side with or without a barrier layer as described above. The matrix 50 portion and matrix and anti-restenotic agent 60 portion are arranged in a programmable manner to achieve a desire release rate and administration period which will be described in further detail below. As can be seen FIG. 4, the concentration of the therapeutic agent (Os) is highest at a center of the stent 10 and lower at the mural side 26 of the stent to achieve a substantially linear release rate with a minimal initial start up release.

Numerous other useful arrangements of the matrix and therapeutic agent can be formed to achieve the substantially linear release, extended release, and substantially complete release described herein. Each of the areas of the matrix may include one or more agents in the same or different proportions from one area to the next. The matrix may be solid, porous, or filled with other drugs or excipients. The agents may be homogeneously disposed or heterogeneously disposed in different areas of the matrix.

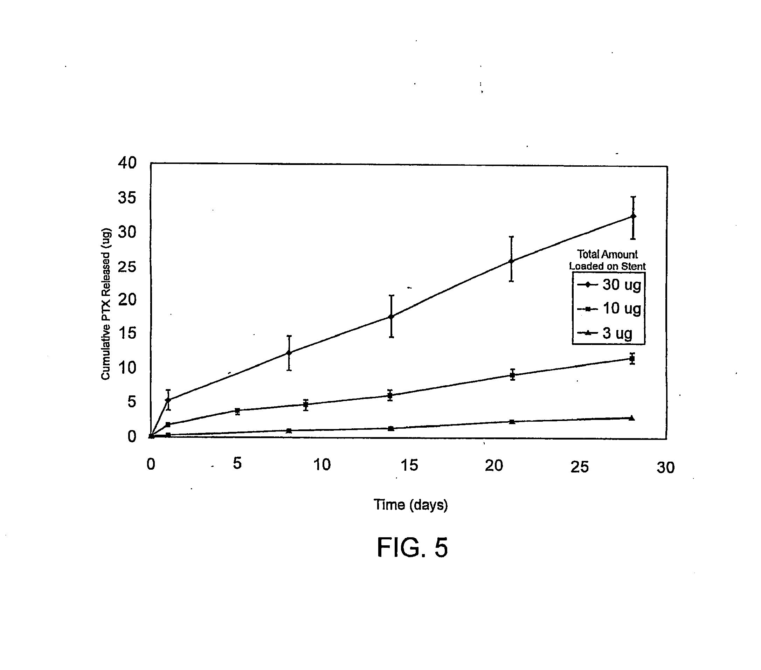

FIG. 5 illustrates three examples of extended-linear drug release profiles which are characterized by a small initial release of drug in the first day, followed by a substantially linear extended release until all the drug loaded on the stent is released. Preferably, the initial release in the first day of administration will be less than 25% of the total drug loaded. In the examples, the drug released is paclitaxel which is loaded in a PLGA matrix for directional delivery to the mural side of the stent The drug release rate is programmed by providing different concentrations of drug in different areas of the matrix.

The method for administering a dose of anti-restenotic agent, such as paclitaxel, can include delivering 2-25% of the total amount of agent loaded into the stent in the first day, then delivering drug in a substantially linear fashion a total 95% of the loaded drug by day 20-45. Following the first day release, the rate of extended substantially linear drug release will be in the range of greater than 1% per day, preferably about 1.5% to about 5% of the total loaded drug dose per day, and more preferably the substantially linear release rate is in the range of about 2% to about 4% of total drug loaded per day.

The release profile for a drug or therapeutic agent can be defined by a plot of the cumulative drug released versus the time during which the release takes place, as shown in FIG. 4. By substantially linear release profile is meant that the linear least squares fit of such a release profile plot has a correlation coefficient value, r², of greater than 0.92 for data time points after the first day of delivery. According to one preferred embodiment, an anti-restenotic, such as paclitaxel is released at a substantially linear release rate in which r² is greater than 0.95 after the first day of delivery with less than 25% of the total drug loaded delivered in the first day.

When the anti-restenotic agent delivered by the method of the invention is paclitaxel, the total amount delivered (and loaded) is preferably between 2 micrograms and 50 micrograms. In one preferred embodiment, the amount of paclitaxel delivered will be between about 0.1 micrograms and about 15 micrograms on the first day, more preferably between about 0.3 micrograms and about 9 micrograms. Following day one, the paclitaxel will be delivered in a substantially linear fashion at a rate of about 0.025 micrograms to about 2.5 microgram per day for a minimum of 21 days, preferably about 0.2 to about 2 micrograms per day. It is envisioned that all the paclitaxel will be released from the stent in less than 60 days. The total amount of paclitaxel loaded onto the stent and released into the tissue in need of treatment is preferably in the range of about 1.5 micrograms to about 75 micrograms, more preferably about 3 to about 30 micrograms, and more preferably about 10 micrograms. The above release rates for paclitaxel have been given for a average stent of dimensions 3.0 mm in expanded diameter by 17 mm in length. For this average 17 mm stent, the release rate is preferably about 0.1 to about 0.5 micrograms per day. Initial indications show that slightly higher dosages (i.e. about 0.5 to about 1.5 micrograms per day) can be advantageous for certain classes of patients, such as diabetics.

Stents of other dimensions will contain total drug loadings in similar respective proportions based on similar drug loading density. In one example, the amount of paclitaxel released per day after day one is about 0.0003 to about 0.03 ug/mm² of tissue surface area, preferably about 0.0003 to about 0.01 ug/mm² of tissue surface area. In another example, the amount of paclitaxel released per day after day one is about 0.001 to about 0.2 ug/mm of stent length per day.

The methods of the invention preferably will result in sustained release of substantially all the drug loaded onto the stent in no longer than 180 days, preferably in no longer than 60 days, and most preferably in no longer than 35 days.

When the anti-restenotic agent is paclitaxel, at least 50% of the paclitaxel loaded into the stent is preferably released and no more than 50% of the amount is non-releasable. Non-releasable paclitaxel is paclitaxel that is sequestered in the polymeric matrix such that it is not released under physiologic conditions is less than 180 days. Preferably, more than 80% of the paclitaxel loaded will be released in no longer than 180 days, more preferably all the paclitaxel will be released.

In one preferred embodiment, agent will be delivered from a polymer matrix reservoir in the stent, where the polymer is a bioresorbable polymer. In the case of a bioresorbable polymer, preferably all of the drug is eluted from the stent before all of the polymer matrix is resorbed. Typically all polymer drug delivery matrix will be bioresorbed in 14 days to one year, more preferably in 30 days to 90 days.

The substantially linear extended drug delivery profiles described above and the examples shown in FIG. 5 can become a zero order release profile, or can be a zero order release profile after the second day of drug delivery.

It has been shown in clinical trials that longer constant or substantially linear release of the anti-restenotic paclitaxel, such as in the release profiles shown in FIG. 5 results in lower in stent neointimal proliferation than the more rapid release of the same dosage. The method of substantially linear extended release of anti-restenotic agents increases the therapeutic effectiveness of administration of a given dose of agent and reduces side effects.

A multi-center study involving 191 patients in nine sites across seven countries, compared the safety and performance of paclitaxel delivered at different rates and doses. Patients participating in the trial received six different formulations of paclitaxel that varied by dose, drug release rate (fast, medium and slow) and directionality (drug release to only the arterial wall vs. release to both the wall and the lumen). Included in the doses where the 10 µg/30 day and the 30 µg/30 day doses listed in FIG. 5. There were 39 patients in the 10 µg dose and 30 patients in the 30 µg dose. The average age was 59.1 + 9.2 and the patients were 70% male.

At four-month follow-up, all six formulations were determined to be safe, with an overall rate of Major Adverse Cardiac Events (MACE) of 5.2 percent. In the 39 patient group receiving a 10 µg, slow release (30-day) formulation delivered to the vessel wall there were zero MACE. With this formulation, there was an in-stent binary restenosis rate of zero percent, with in-stent angiographic late loss of 0.38 millimeters and 7.7 percent volume obstruction determined by Intravascular Ultrasound (IVUS). In addition, the target lesion revascularization (TLR) rate for this group was zero percent, and the target vessel revascularization (TVR) rate was 2.6 percent. By comparison, in another group receiving a fast release (10 day) formulation of 10 ug delivered murally, there was an in-stent restenosis rate of 3.6 percent, in-stent angiographic late loss of 0.67 millimeters, and 17.3 percent volume obstruction by IVUS. These results indicate that the drug release rate has an effect on treatment outcomes and that the extended release over 30 days shows better results than the 10 day release of the same dosage.

In the 30 patient group receiving a 30 µg, slow release (30-day) formulation delivered to the vessel wall there were also zero MACE in the 30 day to 4 month period. With this formulation, there was an in-stent binary restenosis rate of 7.4 percent, with in-stent angiographic late loss of 0.37 millimeters and 5.1 percent volume obstruction determined by Intravascular Ultrasound (IVUS). In addition, the target lesion revascularization (TLR) rate for this group was zero percent, and the target vessel revascularization (TVR) rate was 3.3 percent.

### THERAPEUTIC AGENTS

While the invention has been describe with respect to treatment of restenosis, other therapeutic agents may be delivered at the release profiles described for treatment of other conditions, such as acute myocardial infarction, thrombosis, or for passivation of vulnerable plaque.

The present invention relates to the delivery of anti-restenotic agents including taxol, rapamycin, other limus drugs, cladribine, colchicines, vinca alkaloids, heparin, hinrudin and their derivatives, as well as other cytotoxic or cytostatic agents and microtubule stabilizing and microtubule inhibiting agents. Although anti-restenotic agents have been primarily described herein, the present invention may also be used to deliver other agents alone or in combination with anti-restenotic agents.

Other therapeutic agents for use with the present invention may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, paclitaxel, actinomycin D, rapamycin, and cyclosporin.

Antithrombins include, without limitation, heparin, plasminogen, α₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dihydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation, glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limitation, Adiponectin.

While the invention has been described in detail with reference to the preferred embodiments thereof, it will be apparent to one skilled in the art that various changes and modifications can be made and equivalents employed, without departing from the present invention.

The subject matter described in the following paragraphs, numbered for reference, is part of the disclosure of the present application, each of which can be claimed in the present application, and in one or more future divisional applications there from:
(1). A stent for reducing restenosis comprising: a drag delivery stent having initial unexpanded diameter for insertion of the stent into a coronary artery and an expanded diameter for implantation within a coronary artery, the stent having a dosage of paclitaxel for delivery to an artery, the dosage arranged such that substantially all the paclitaxel is releasable from the stent upon implantation of the stent in the artery, wherein the dosage of paclitaxel is arranged to be released at a minimum release rate of 1 percent of the total dosage of paclitaxel on the stent per day throughout an entire administration period from the time of implantation of the stent until the time that substantially all the paclitaxel is released from the stent.
(2). The stent of paragraph 1 above, wherein the administration period is about 20 to about 40 days from the date of implantation.
(3). The stent of paragraph 1 above, wherein the release rate of the paclitaxel after day one is substantially linear.
(4). The stent of paragraph 3 above, wherein the substantially linear release rate is defined as a release rate in which r2 is greater than 0.95.
(5). The stent of paragraph 1 above, wherein the amount of paclitaxel released per day after day one is about 0.0003 to about 0.03 ug/mm2 of tissue surface area.
(6). The stent of paragraph 1 above, wherein the paclitaxel is deposited in openings in the stent.
(7). The stent of paragraph 1 above, wherein the paclitaxel is contained in a bioresorbable matrix.
(8). The stent of paragraph 1 above wherein the paclitaxel is contained in a polymer matrix.
(9). The stent of paragraph 1 above, wherein the paclitaxel is arranged to be delivered primarily murally from the stent.
(10). The stent of paragraph 1 above, wherein the paclitaxel is affixed to the stent such that 2-25% of the total amount of paclitaxel loaded into the stent is delivered in the first day, 95% of the loaded paclitaxel delivered by day 20 to 45.
(11). The stent of paragraph 1 above, wherein the paclitaxel is loaded for delivery after day one at a rate of about 0.25 micrograms to about 2.5 microgram per day for a minimum of 21 days for a stent with dimensions 3.0 mm in expanded diameter by 17 mm in length, and delivering other amounts from stents of other dimensions based on their respective relative proportions.
(12). The stent of paragraph 1 above, wherein the paclitaxel is affixed to the stent such that more than 80% of the paclitaxel loaded on the stent is delivered in no longer than 180 days.
(13). The stent of paragraph 1 above, wherein the amount of paclitaxel released per day after day one is about 0.1 to about 0.5 µg.
(14). A method of reducing restenosis comprising: providing a drug delivery stent having a dosage of paclitaxel for delivery to an artery, the dosage arranged such that substantially all the paclitaxel is releasable from the stent upon implantation of the stent in the artery; implanting the stent within an artery of a patient; and delivering paclitaxel from the stent to the artery at a minimum release rate of 1 percent of the total dosage of paclitaxel on the stent per day throughout an entire administration period from the time of implantation of the stent until the time that substantially all the paclitaxel is released from the stent.
(15). The method of paragraph 14 above, wherein the administration period is about 20 to about 40 days from the date of implantation.
(16). The method of paragraph 14 above, wherein the release profile of the paclitaxel after day one is substantially linear.
(17). The method of paragraph 14 above, wherein the amount of paclitaxel released per day after day one is about 0.0003 to about 0.03 ug/mm2 of tissue surface area.
(18). The method of paragraph 14 above, wherein the paclitaxel is deposited in openings in the stent.
(19). The method of paragraph 14 above, wherein the paclitaxel is contained in a bioresorbable matrix.
(20). The method of paragraph 14 above, wherein the paclitaxel is contained in a polymer matrix.
(21). The method of paragraph 14 above, wherein the paclitaxel is delivered primarily murally from the stent.
(22). The method of paragraph 14 above, wherein the step of delivering paclitaxel further comprises delivering 2-25% of the total amount of paclitaxel loaded into the stent in the first day, then delivering 95% of the loaded paclitaxel by day 20 to 45.
(23). The method of paragraph 14 above, wherein the step of delivering paclitaxel further comprises delivering paclitaxel after day one at a rate of about 0.25 micrograms to about 2.5 microgram per day for a minimum of 21 days for a stent with dimensions 3.0 mm in expanded diameter by 17 mm in length, and delivering other amounts from stents of other dimensions based on their respective relative proportions.
(24). The method of paragraph 14 above, wherein the step of delivering paclitaxel further comprises delivering more than 80% of the paclitaxel loaded on the stent in no longer than 180 days.
(25). A method of reducing restenosis comprising: providing a drug delivery stent having a dosage of paclitaxel for delivery to an artery; implanting the stent within an artery of a patient; and delivering paclitaxel from the stent to the artery at a substantially linear release rate over an entire period from day one after implantation through day twenty five after implantation, wherein the amount of paclitaxel delivered during the period is at least 25% of the drug loaded on the stent.
(26). The method of paragraph 25 above, wherein the amount of paclitaxel released per day after day one is about 0.0003 to about 0.03 ug/mm2 of tissue surface area.
(27). The method of paragraph 25 above, wherein the paclitaxel is deposited in openings in the stent.
(28). The method of paragraph 25 above, wherein the paclitaxel is contained in a bioresorbable polymer matrix.
(29). The method of paragraph 25 above, wherein the paclitaxel is delivered primarily murally from the stent.
(30). The method of paragraph 25 above, wherein the step of delivering paclitaxel further comprises delivering 2-25% of the total amount of paclitaxel loaded into the stent in the first day, then delivering 95% of the loaded paclitaxel by day 20 to 45.
(31). The method of paragraph 25 above, wherein the step of delivering paclitaxel further comprises delivering more than 80% of the paclitaxel loaded on the stent in no longer than 30 days.
(32). A method of reducing restenosis comprising: providing a drug delivery stent having a dosage of paclitaxel for delivery to an artery; implanting the stent within an artery of a patient; and delivering paclitaxel from the stent to the artery, wherein at least 80% of the entire dosage of paclitaxel provided by the stent is delivered to the artery within 60 days of implantation.
(33). The method of paragraph 32 above, wherein the release profile of the paclitaxel after day one is substantially linear.
(34). The method of paragraph 32 above, wherein the amount of paclitaxel released per day after day one is about 0.0003 to about 0.03 ug/mm2 of tissue surface area.
(35). The method of paragraph 32 above, wherein the paclitaxel is deposited in openings in the stent.
(36). The method of paragraph 32 above, wherein the paclitaxel is contained in a bioresorbable polymer matrix.
(37). The method of paragraph 32 above, wherein the paclitaxel is delivered primarily murally from the stent.
(38). The method of paragraph 32 above, wherein the step of delivering paclitaxel further comprises delivering paclitaxel after day one at a rate of about 0.25 micrograms to about 2.5 microgram per day for a minimum of 21 days for a stent with dimensions 3.0 mm in expanded diameter by 17 mm in length, and delivering other amounts from stents of other dimensions based on their respective relative proportions.
(39). A method of reducing restenosis comprising: providing a drug delivery stent having a dosage of an anti-restenotic drug for delivery to an artery, the dosage arranged such that substantially all the drug is releasable from the stent upon implantation of the stent in the artery; implanting the stent within an artery of a patient; and delivering the drug from the stent to the artery at a minimum release rate of 1 percent of the total dosage of the drug on the stent per day throughout an entire administration period from the time of implantation of the stent until the time that substantially all the drug is released from the stent, wherein the release rate of the drug is substantially linear from at least day two through day 25.
(40). The method of paragraph 39 above, wherein the administration period is about 20 to about 40 days from the date of implantation.
(41). The method of paragraph 39 above, wherein the drug is deposited in openings in the stent.
(42). The method of paragraph 39 above, wherein the drug is contained in a bioresorbable polymer matrix.
(43). The method of paragraph 39 above, wherein the drug is delivered primarily murally from the stent.
(44). The method of paragraph 39 above, wherein the step of delivering drug further comprises delivering 2-25% of the total amount of drug loaded into the stent in the first day, then delivering 95% of the loaded drug by day 20 to 45.
(45). The method of paragraph 14 above, wherein the step of delivering drug further comprises delivering more than 80% of the drug loaded on the stent in no longer than 180 days.
(46). The method of paragraph 14 above, wherein the step of delivering drug further comprises releasing the drug at a substantially linear release rate in which r2 is greater than 0.95 after the first day of delivery and with less than 25% of the total drug loaded delivered in the first day.
(47). A method of treating a patient comprising: providing a drug delivery stent having a dosage of therapeutic agent for delivery to an artery, the dosage arranged such that substantially all the agent is releasable from the stent upon implantation of the stent in the artery; implanting the stent within an artery of a patient; and delivering the agent from the stent to the artery at a minimum release rate of 1 percent of the total dosage of the agent on the stent per day throughout an entire administration period from the time of implantation of the stent until the time that substantially all the drug is released from the stent, wherein the release rate of the drug after day one is substantially linear from at least day 2 through day 25.
(48). The method of paragraph 47 above, wherein the administration period is about 20 to about 40 days from the date of implantation.
(49). The method of paragraph 47 above, wherein the drug is deposited in openings in the stent.
(50). The method of paragraph 47 above, wherein the drug is contained in a bioresorbable polymer matrix.
(51). The method of paragraph 47 above, wherein the step of delivering drug fUrther comprises releasing the drug at a substantially linear release rate in which r2 is greater than 0.95 after the first day of delivery and with less than 25% of the total drug loaded delivered in the first day.

## Claims

1. An anti-restenotic drug to be delivered from a stent loaded with a predetermined amount of the anti-restenotic drug for use in a method of reducing restenosis, the method comprising implanting the stent having no more than the predetermined amount of the anti-restenotic drug; and delivering the anti-restenotic drug from a bioresorbable polymer matrix reservoir in the stent,
wherein the polymer matrix is completely resorbed between 30 days and 90 days, inclusive, and the entire predetermined amount elutes from the stent before the polymer matrix is resorbed.

2. The drug as in claim 1, wherein the anti-restenotic drug is rapamycin.

3. The drug as in claim 1, wherein the anti-restenotic drug is paclitaxel.

4. The drug as in one of claims 1 to 3, wherein the delivery profile after implantation is substantially linear extended release.

5. The drug as in one of claims 1 to 4, wherein the stent is arranged for delivery primarily murally.

6. The drug as in one of claims 1 to 5, wherein the bioresorbable polymer matrix includes polylactic acid PLA.

7. The drug as in one of claims 1 to 6, wherein the bioresorbable polymer matrix includes polylactic-co-glycolic acid PLGA.
